# EUROPÄISCHE PATENTSCHRIFT

(11) **EP 0 748 236 B1**
(45) Veröffentlichungstag und Bekanntmachung des Hinweises auf die Patenterteilung: **27.03.2002**
(21) Anmeldenummer: 95943232.9
(22) Anmeldetag: 29.12.1995
(51) Int. Cl.: A61M 5/36

(54) **INFUSIONSSCHLAUCH FÜR EIN INFUSIONSGERÄT MIT EINEM BLASENDETEKTOR**
INFUSION TUBE FOR AN INFUSION DEVICE WITH A BUBBLE DETECTOR
FLEXIBLE POUR APPAREIL DE PERFUSION, MUNI D'UN DETECTEUR DE BULLES

(30) Priorität: 04.01.1995 DE 19500154
(43) Veröffentlichungstag der Anmeldung: 18.12.1996
(73) Patentinhaber: Fritz Giebler GmbH, 86405 Meitingen (DE)
(72) Erfinder: GIEBLER, Fritz, D-86405 Meitingen (DE)
(74) Vertreter: Feldkamp, Rainer, Dipl.-Ing.
(86) Internationale Anmeldenummer: EP9505168
(87) Internationale Veröffentlichungsnummer: WO9620746

(56) Entgegenhaltungen:
- EP-A- 0 053 453
- EP-A- 0 524 605
- US-A- 4 237 720
- US-A- 5 205 153

## Beschreibung

Die Erfindung bezieht sich auf einen Infusionsschlauch für ein Infusionsgerät mit Blasendetektor der im Oberbegriff des Anspruchs 1 genannten Art.

Bei medizinischen Anwendungen von Infusionsgeräten wird die Flüssigkeitssäule in dem Ihfusionsschlauch auf Einschlüsse von Luftblasen hin überwacht, da derartige Luftblasen nicht in die Blutbahn des Patienten gelangen dürfen.

Obwohl für die Überwachung der Flüssigkeitssäule auf den Einschluß von Luftblasen vielfältige Möglichkeiten zur Verfügung stehen, wird die Anwendung von Ultraschallsensoren bevorzugt.

Ein Verfahren sowie eine Vorrichtung zur Durchführung dieses Verfahrens sind beispielsweise in der DE-OS 3141576 beschrieben. Hierbei sind Ultraschallsenderplättchen und ein Ultraschallempfängerplättchen auf den einander zugewandten Innenseiten der freien Schenkel eines U-förmigen Trägerkörpers angeordnet, wobei der Abstand zwischen Ultraschallsenderplättchen und Ultraschallempfängerplättchen so bemessen ist, daß der Schlauch bzw. die Leitung unter Deformation des kreisförmigen Querschnittes zwischen diesen Plättchen eingeklemmt wird. Abhängig davon, ob sich im Bereich des Schallsignals Flüssigkeit oder Luft im Infusionsschlauch befindet, ist das von dem Ultraschall-Empfänger empfangene Signal größer oder kleiner, so daß das elektrische Ausgangssignal des Ultraschall-Empfängers ausgewertet und zur Feststellung von Lufteinschlüssen ausgenutzt werden kann.

Um die direkte Übertragung von Ultraschallsignalen zwischen diametral zu dem Schlauchabschnitt angeordneten UltraschallSendern und Ultraschall-Empfängern zu verringern, ist es weiterhin aus der EP-A1 0 053 453 bekannt, in einem Kunststoffkörper, der den Ultraschall-Sender und den Ultraschall-Empfänger trägt, einen Luftspalt zwischen dem Sender und dem Empfänger anzuordnen, um die Übertragung von Ultraschallenergie durch den den Sender und Empfänger tragenden Gehäuseskörper zu verringern.

Unerwünschterweise wird das eingestrahlte Schallsignal jedoch nicht nur von dem den Sender und Empfänger tragenden Gehäuse sowie von der Flüssigkeitssäule bzw. von den Lufteinschlüssen weitergeleitet, sondern auch von dem Material des Schlauchabschnittes, der sich zwischen den freien Schenkeln des U-förmigen Trägerkörpers befindet, so daß das Signal-/Störverhältnis gering ist und ggf. nicht einwandfrei reproduzierbare Ergebnisse erzielt werden.

Dieses Problem wird noch dadurch verstärkt, daß in vielen Fällen zur Erzielung eines guten Kontaktes mit den Ultraschall-Sendern bzw. -Empfängern und zur Festlegung der Lage des Schlauches über diesen Infusionsschlauch ein Kunststoffkörper gezogen wird, der exakt in ein Gegenstück im Infusionsgerät paßt. Dieser Kunststoffkörper kann auch ein Verbindungsnippel mit besonderer Form sein, der zwei Anschlußstutzen aufweist, an den zwei Schläuche mit unterschiedlicher Qualität angeschlossen werden, insbesondere dann, wenn ein Infusionsgerät mit einer Rollenpumpe verwendet wird, bei der das in der Rollenpumpe befindliche Schlauchsegment aus Silikonschlauch besteht, während für den übrigen Teil des Infusionsschlauches ein kostengünstigeres Material verwendet wird.

Obwohl dieser Kunststoffkörper ein einwandfreies Anlegen der Ultraschall-Sender bzw. -Empfänger ermöglicht, leitet er sehr stark das Schallsignal unabhängig von dem Schlauch weiter, so daß das Signal-/Störverhältnis weiter verschlechtert wird.

5 Bei Verwendung von Kunststoffkörpern als Teil eines Schlauchabschnittes ist es weiterhin aus der US-A-5 205 153 bekannt, diesen Kunststoffkörper mit sich in Umfangsrichtung erstreckenden Rippen versehen, die eine Konzentration der Ultraschallstrahlung auf mehrere in Axialrichtung hintereinander liegende Bereiche des Schlauchabschnittes bewirkt. Eine Verringerung der Übertragung der Schallsignale in Umfangsrichtung wird hierdurch jedoch nicht erreicht.

Weiterhin ist es bei Verwendung von in Axialrichtung hintereinander liegenden Reflexions-Ultraschallwandlern bekannt, diese Ultraschallwandler durch in den Kunststoffkörper angeordnete Bohrungen am Umfang des jeweiligen Wandlers akustisch voneinander zu isolieren, doch wird auch hier die direkte Schallweiterleitung und Reflexion an den Innenflächen des Kunststoffkörpers nicht verringert.

Der Erfindung liegt die Aufgabe zugrunde, einen Infusionsschlauch für ein Infusionsgerät mit Blasendetektor der eingangs genannten Art zu schaffen, der eine verbesserte Feststellung von Lufteinschlüssen in der Flüssigkeitssäule ermöglicht.

Diese Aufgabe wird durch die im Anspruch 1 angegebenen Merkmale gelöst.

Vorteilhafte Ausgestaltungen und Weiterbildungen der Erfindung ergeben sich aus den Unteransprüchen.

Durch die erfindungsgemäße Ausgestaltung des Infusionsschlauches wird eine definierte Übertragung des Schallsignals an die Flüssigkeitssäule bzw. das Innere des Infusionsschlauches erzielt, so daß sich reproduzierbare Ergebnisse mit hohem Signal-/Störverhältnis erzielen lassen, die einwandfrei reproduzierbar sind, da die Kontaktelemente und die Filter- und/oder Dämpfungsglieder durch einfaches Abformen eines Kunststoffteils sowohl kostengünstig als auch in genau reproduzierbarer Weise herzustellen sind.

Der Kunststoffkörper ist vorzugsweise als Verbindungsnippel zum Verbinden von zwei Schlauchabschnitten des Infusionsschlauches aus unterschiedlichem Material ausgebildet, oder er kann mit engem Sitz auf einen Infusionsschlauch aufgezogen werden.

Im Inneren des Kunststoffkörpers können weiterhin zusätziche Meßelemente ageordnet sein, beispielsweise ein Flügelrad, das zur Überwachung der Strömungsgeschwindigkeit dient, wobei die Bewegungsbahn dieses beweglichen Körpers das Schallsignal schneidet und somit die Bewegung dieses beweglichen Elementes in dem elektrischen Ausgangssignal des Ultraschall-Empfängers abzuleiten ist.

Die Erfindung wird im folgenden anhand von in der Zeichnung dargestellten Ausführungsbeispielen noch näher erläutert.

In der Zeichnung zeigen:
Fig. 1 eine Schnittansicht einer ersten Ausführungsform eines Abschnittes des Infusionsschlauches,
Fig. 2 eine Seitenansicht des Abschnittes des Infusionsschlauches nach Fig. 1,
Fig. 3 eine zweite Ausführungsform eines Abschnittes des Infusionsschlauches,
Fig. 4 eine Querschnittsansicht des Abschnittes nach Fig. 3.

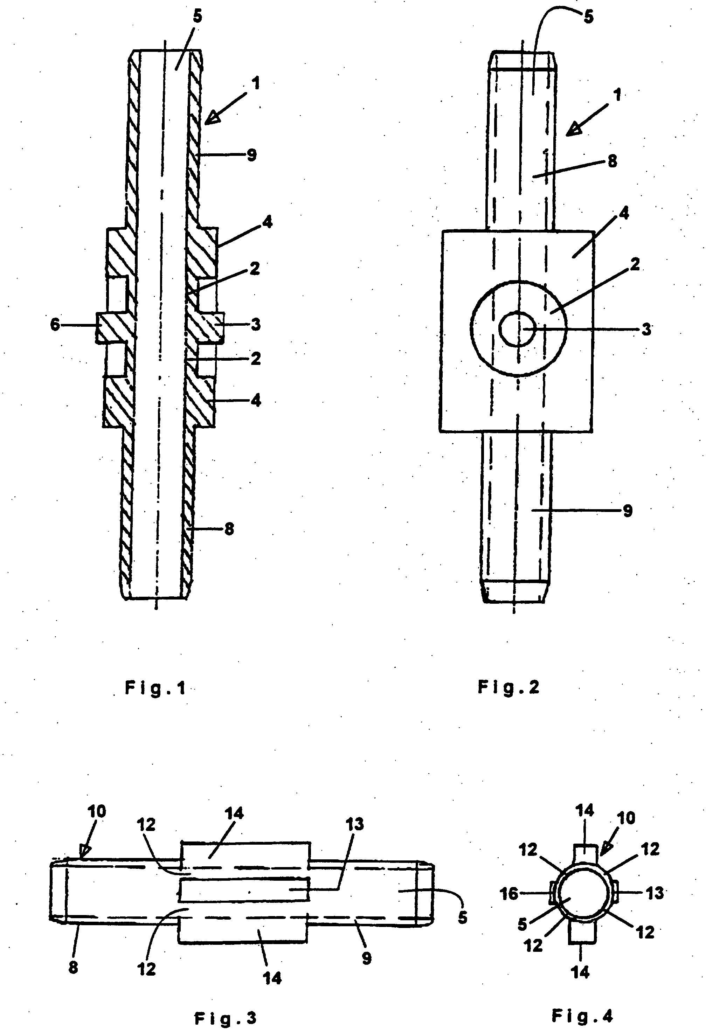

Die in den Fig. 1 und 2 dargestellte erste Ausführungsform des Schlauchabschnittes ist in Form eines langgestreckten Kunststoffkörpers 1 ausgebildet, der an seinen beiden axialen Enden jeweils Schlauchstutzen 8, 9 aufweist, die zum Aufschieben von jeweiligen weiteren Segmenten des Infusionsschlauches dienen, die aus unterschiedlichem Material bestehen können. Der Kunststoffkörper 1 weist einen durchgehenden Flüssigkeitskanal 5 auf, in den selbstverständlich auch ein durchgehender Infusionsschlauch mit engem Sitz eingeschoben werden kann.

Im Mittelbereich des Kunststoffkörpers 1 ist ein verstärkter Kunststoffblock 4 vorgesehen, in dem auf diametral gegenüberliegenden Seiten des Flüssigkeitskanals 5 Kontaktelemente 3, 6 ausgebildet sind, die über einen kreisringförmigen Bereich 2 mit geringerem Querschnitt und damit geringerer Masse mit dem übrigen Teil des Kunststoffblockes 4 verbunden sind. Diese Bereiche 2 mit verringerten Querschnitt und damit verringerter Masse bilden ein akustisches Filterelement und isolieren die Kontaktelemente 3, 6 von dem übrigen Teil des Kunststoffblockes 4, so daß ein beispielsweise in das Kontaktelement 3 eingespeistes Schallsignal sich nur in geringem Umfang in Radialrichtung der geschwächten Bereiche 2 nach außen hin ausbreitet und der verbleibende Teil des Schallsignals endgültig in dem umgebenden Bereich 4 des Kunststoffblockes abgedämpft und absorbiert wird. Auf diese Weise sind reproduzierbare Verhältnisse erzielbar, und die mit den freien Enden der Kontaktelemente 3, 6 gekoppelten Ultraschall-Sender und -Empfänger weisen eine einwandfreie Kopplung mit der in dem Flüssigkeitskanal 5 befindlichen Flüssigkeitssäule auf.

In den Fig. 3 und 4 ist eine weitere Ausführungsform des Abschnittes des Infusionsschlauches dargestellt, wobei auch diese Ausführungsform einen Kunststoffkörper 10 aufweist, der mit zwei Anschlußstutzen 8, 9 zur Verbindung mit jeweiligen Segmenten des Infusionsschlauches versehen ist.

Dieser Kunststoffkörper 10 weist ebenfalls einen ihn axial durchlaufenden Strömungskanal 5 auf. Um diesen Strömungskanal herum sind im Mittelbereich des Kunststoffkörpers 1 einander abwechselnde langgestreckte Vorsprünge 13, 16 mit geringerer Masse und Vorsprünge 14 mit höherer Masse angeordnet, die in Umfangsrichtung durch Bereiche 12 mit geringerer Masse voneinander getrennt sind. Diese Bereiche 12 ergeben eine Isolation und Filterwirkung, während die Vorsprünge 14 eine Dämpfung ergeben, so daß in die Vorsprünge 13, 16 eingekoppelte bzw. aus diesen ausgekoppelte Schallsignale sehr stark von dem übrigen Teil des Kunststoffkörpers 10 isoliert sind und die eine größere Masse aufweisenden Vorsprünge 14 verbleibende Schallschwingungen dämpfen.

Obwohl dies nicht dargestellt ist, ist es selbstverständlich möglich, im Inneren der Kunststoffkörper 1 bzw. 10 bewegliche Körper anzuordnen, beispielsweise Schwebekörper, die durch die Flüssigkeitsströmung ausgelenkt werden, oder ein Flügelrad, das durch die Flüssigkeitsströmung angetrieben wird, wobei die Bewegungsbahn dieser beweglichen Körper die Bahn des Schallsignals schneidet und dieses Schallsignal damit gleichzeitig zur Strömungsmessung herangezogen werden kann.

## Patentansprüche

1. Infusionsschlauch für ein Infusionsgerät mit einem Blasendetektor, bei dem zumindest in einem Abschnitt des Infusionsschlauches dauernd eine Flüssigkeitssäule aufrechterhalten wird und bei dem benachbart zu diesem Abschnitt Ultraschall-Detektoreinrichtungen angeordnet sind, die einen Ultraschall-Sender bzw. -Empfänger aufweisen, deren Schallsignal die Flüssigkeitssäule in dem Abschnitt des Infusionsschlauches diametral durchläuft und zur Feststellung von Lufteinschlüssen in der Flüssigkeitssäule dient, **dadurch gekennzeichnet, daß** an dem Abschnitt des Infusionsschlauches im wesentlichen diametral gegenüberliegende Kontaktelemente (3, 6; 13, 16) für einen Kontakt mit dem Ultraschall-Sender bzw. -Empfänger ausgebildet sind und daß die Kontaktelemente (3, 6; 13, 16) durch akustische Filter- und/oder Dämpfungsglieder (2, 4; 12, 14) von dem umgebenden Bereich des Abschnittes des Infusionsschlauches isoliert sind.

2. Infusionsschlauch nach Anspruch 1, **dadurch gekennzeichnet, daß** die akustischen Filter- und/oder Dämpfungsglieder durch einen das jeweilige Kontaktelement (3, 6; 13, 16) umgebenden Bereich (2; 12) geringerer Masse und einen diesen Bereich geringerer Masse umgebenden Bereich (4; 14) vergrößerter Masse gebildet sind.

3. Infusionsschlauch nach Anspruch 1 oder 2, **dadurch gekennzeichnet, daß** der Abschnitt des Infusionsschlauches durch einen Kunststoffkörper (1) gebildet ist, in dem Kontaktelemente (3, 6; 13, 16) und die akustischen Filter- und/oder Dämpfungsglieder (2, 4; 12, 14) ausgeformt sind.

4. Infusionsschlauch nach Anspruch 3,
**dadurch gekennzeichnet, daß** der Kunststoffkörper (1) auf einen elastischen Infusionsschlauch aufgeschoben ist.

5. Infusionsschlauch nach Anspruch 3 oder 4, **dadurch gekennzeichnet, daß** der Kunststoffkörper (1) einen Strömungskanal (5) aufweist und daß an den Enden des Kunststoffkörpers Anschlußstutzen (8, 9) ausgebildet sind, auf die jeweilige Schlauchenden von weiteren Abschnitten des Infusionsschlauches aufschiebbar sind.

6. Infusionsschlauch nach einem der Ansprüche 3 bis 5,
**dadurch gekennzeichnet, daß** in dem Kunststoffkörper (1) ein bewegliches Element angeordnet ist, dessen Bewegungsbahn die Bahn des Schallsignals durchquert.

7. Infusionsschlauch nach Anspruch 6, **dadurch gekennzeichnet, daß** das bewegliche Element ein Flügelrad zur Messung der Strömungsgeschwindigkeit ist.

## Claims

1. Infusion tube for an infusion unit with a bubble detector, wherein a column of liquid is permanently maintained at least in one section of the infusion tube and wherein adjacent to said section there are arranged ultrasound detector devices which incorporate an ultrasound transmitter and receiver, respectively, the acoustic signal from which passes diametrically through the column of liquid in the section of the infusion tube and is used for detecting air inclusions in the column of liquid,
**characterised in that** formed on the section of the infusion tube are substantially diametrically opposed contact elements (3, 6; 13, 16) for a contact with the ultrasound transmitter or receiver, respectively, and that the contact elements (3, 6; 13, 16) are insulated from the surrounding region of the section of infusion tube by acoustic filters and/or attenuators (2, 4; 12, 14).

2. Infusion tube according to claim 1, **characterised in that** the acoustic filters and/or attenuators are constituted by a region (2; 12) of lesser mass which surrounds the respective contact element (3, 6; 13, 16) and by a region (4; 14) of greater mass which surrounds this region of lesser mass.

3. Infusion tube according to claim 1 or 2,
**characterised in that** the section of infusion tube is constituted by a plastics body (1) in which contact elements (3, 6; 13, 16) and the acoustic filter elements and/or attenuators (2, 4; 12, 14) are incorporated.

4. Infusion tube according to claim 3,
**characterised in that** the plastics body (1) is pushed onto a flexible infusion tube.

5. Infusion tube according to claim 3 or 4,
**characterised in that** the plastics body (1) incorporates a flow channel (5) and that configured at the extremities of the plastics body are tube fittings (8, 9) onto which the respective tube ends of further sections of the infusion tube can be pushed.

6. Infusion tube according to any of claims 3 to 5,
**characterised in that** arranged in the plastics body (1) is a moving element whose path of travel crosses the path of the acoustic signal.

7. Infusion tube according to claim 6, **characterised in that** the moving element is an impeller wheel for measuring the rate of flow.

## Revendications

1. Flexible de perfusion pour un appareil de perfusion, doté d'un détecteur de bulles, dans lequel est constamment conservée au moins dans un segment du flexible de perfusion une colonne de liquide et dans lequel sont disposés des dispositifs de détecteurs d'ultrasons à proximité de ce segment, qui comportent un émetteur d'ultrasons et/ou un récepteur d'ultrasons, dont le signal sonore traverse diamétralement la colonne de liquide dans le segment du flexible de perfusion et qui sert à la détection d'inclusions d'air dans la colonne de liquide, **caractérisé en ce que** des éléments de contact (3, 6; 13, 16) essentiellement diamétralement opposés sont disposés au niveau du segment du flexible de perfusion, pour un contact avec l'émetteur et/ou le récepteur d'ultrasons, et **en ce que** les éléments de contact (3, 6; 13, 16) sont isolés de la zone environnante du segment de flexible de perfusion par des éléments de filtrage et/ou d'atténuation acoustiques (2, 4 ; 12, 14).

2. Flexible de perfusion selon la revendication 1, **caractérisé en ce que** les éléments de filtrage et/ou d'atténuation acoustiques sont formés par une zone (2 ; 12) entourant l'élément de contact respectif (3, 6 ; 13, 16) de moindre masse et par une zone de masse supérieure (4 ; 14) entourant cette zone de moindre masse.

3. Flexible de perfusion selon la revendication 1 ou 2, **caractérisé en ce que** le segment du flexible de perfusion est formé par un corps de plastique (1), dans lequel sont formés des éléments de contact (3, 6; 13, 16) et les éléments de filtrage et/ou d'atténuation acoustiques (2, 4 ; 12, 14).

4. Flexible de perfusion selon la revendication 3, **caractérisé en ce que** le corps de plastique (1) est enfilé sur un flexible de perfusion élastique.

5. Flexible de perfusion selon la revendication 3 ou 4, **caractérisé en ce que** le corps de plastique (1) comporte un canal d'écoulement (5) et **en ce que** sont conçus au niveau des extrémités du corps de plastique des raccords de tuyaux (8, 9), sur lesquels peuvent être insérées les extrémités de flexible respectives d'autres segments du flexible de perfusion.

6. Flexible de perfusion selon l'une des revendications 3 à 5, **caractérisé en ce qu'**un élément mobile est disposé dans le corps de plastique (1), dont la trajectoire de déplacement coupe la trajectoire de déplacement du signal sonore.

7. Flexible de perfusion selon la revendication 6, **caractérisé en ce que** l'élément mobile est une roue hélice pour mesurer la vitesse de l'écoulement.
